# EUROPÄISCHE PATENTSCHRIFT

(11) **EP 0 694 287 B1**
(45) Veröffentlichungstag und Bekanntmachung des Hinweises auf die Patenterteilung: **20.09.2000**
(21) Anmeldenummer: 95111971.8
(22) Anmeldetag: 28.07.1995
(51) Int. Cl.: A61B 17/00, F04B 43/12

(54) **Schlauchpumpe zur genauen Dosierung kleiner Flüssigkeitsmengen**
Hose pump for dosing small amounts of liquid
Pompe à tuyau pour le dosage exacte de petites quantités de liquides

(30) Priorität: 28.07.1994 DE 9412228 U
(43) Veröffentlichungstag der Anmeldung: 31.01.1996
(73) Patentinhaber: Loctite Deutschland GmbH, 81925 München (DE)
(72) Erfinder: Rauh, Erwin, D-85586 Poing (DE)
(74) Vertreter: Abitz, Walter, Dr.-Ing.

(56) Entgegenhaltungen:
- GB-A- 2 076 068
- US-A- 3 990 444
- US-A- 4 214 681
- US-A- 4 660 607
- IBM TECHNICAL DISCLUSURE BULLETIN, Bd. 20, Nr. 3, 3.August 1977 Seiten 987-988, J. H. WILLIAMS 'PERISTALTIC ROLLER PUMP'

## Beschreibung

Die Erfindung betrifft eine Schlauchpumpe zur genauen Dosierung kleiner Flüssigkeitsmengen (vgl. z.B. GB-A-2 076 068). Die Schlauchpumpe enthält ein Schlauchbett und eine Anzahl Rollen, die auf einer drehangetriebenen Rollenaufnahme drehbar auf einem Teilkreis der Rollenaufnahme gelagert sind und durch die Rollenaufnahme auf einer Kreisbahn im Abstand von dem Schlauchbett bewegt werden, wobei der Abstand so gewählt ist, daß die Rollen einen Schlauch, der zwischen dem Schlauchbett und der Kreisbahn der Rollen angeordnet wird, auf einem Teil ihrer Kreisbahn zusammendrücken können.

Derartige Schlauchpumpen sind allgemein bekannt. Sie werden insbesondere auf dem medizinischen Sektor, z.B. für die Dialyse und bei Herz-Lungen-Maschinen eingesetzt, da die zu pumpende Flüssigkeit, hier also das Blut, nur mit der Schlauchwandung und nicht mit anderen Oberflächen in Berührung kommt. Schlauchpumpen sind mechanisch sehr einfach aufgebaut. Mit den bekannten Schlauchpumpen ist eine genaue Dosierung der Größe einzeln ausgegebener Tropfen nicht möglich. Dies ist darauf zurückzuführen, daß die Flüssigkeit stoßweise entsprechend der Frequenz der von den Rollen auf den Schlauch ausgeübten Druckimpulse gepumpt wird.

Der Erfindung liegt die Aufgabe zugrunde, die Dosierbarkeit der Förderleistung einer Schlauchpumpe zu verbessern und eine gleichmäßige Förderung der Flüssigkeit zu erreichen.

Erfindungsgemäß wird diese Aufgabe dadurch gelöst, daß das Schlauchbett versetzt gegenüber der Kreisbahn der Rollen angeordnet ist, so daß der Abstand zwischen Mulde und Rollen-Kreisbahn in Bewegungsrichtung der Rollen zunimmt.

Vorzugsweise ist das Schlauchbett etwa um den Innendurchmesser des Schlauches gegenüber der Rollenkreisbahn versetzt.

Dadurch, daß der Abstand zwischen der Mulde des Schlauchbettes und der Rollen-Kreisbahn in Förderrichtung zunimmt, liegt der Punkt des maximalen Anpreßdruckes außermittig, d.h. er ist von der Mitte der Mulde entgegen der Förderrichtung verschoben. Im allgemeinen liegt der Punkt des maximalen Anpreßdruckes auf den Schlauch am Anfang der Mulde und öffnet sich der Schlauch durch den größer werdenden Abstand zwischen Mulde und Rollen-Kreisbahn keilförmig. Dadurch werden die Pumpstöße gedämpft und wird ein nahezu kontinuierliches Pumpverhalten erzielt.

Die erfindungsgemäße Schlauchpumpe eignet sich insbesondere zur Ausgabe kleinster Flüssigkeitsmengen, z.B. zur reproduzierbaren Ausgabe einzelner Tropfen eines Klebstoffes. Für die Ausgabe jedes Tropfens wird die Rollenaufnahme dabei um einen bestimmten Schrittwinkel weiter gedreht. Die Ausbringmenge, d.h. die Tropfengröße, hängt in erster Linie vom Schrittwinkel ab, den die Rollenaufnahme für diese Ausbringmenge ausführt. Zweckmäßig werden die Teilung der Rollenaufnahme und der Schrittwinkel so gewählt, daß der Schrittwinkel nicht gleich der Teilung der Rollenaufnahme (360°/Anzahl der Rollen), einem Bruchteil oder einem Vielfachen davon ist.

Bei Festlegung der Rollenzahl ist außerdem darauf zu achten, daß das mechanische System der Schlauchpumpe und das elektrische System des Antriebsmotors soweit wie möglich entkoppelt werden, um ein ungünstiges Schwingungsverhalten zu vermeiden. Für den Schrittwinkel ergibt sich dadurch folgende Bedingung:
Schrittwinkel ≠ 2k* 360°/n
n = Anzahl der Rollen
k = 1/m ...1...i
m ∈ ∞..2 ; i ∈ 2..∞

Aus dieser Beziehung ergibt sich, daß eine ungerade Rollenanzahl zweckmäßiger ist, da bei ihr weit weniger Systemzustände auszugrenzen sind als bei einer geraden Rollenzahl, um periodische Vorgänge im Volumenstrom zu vermeiden.

Bei der Festlegung der Rollenzahl und der Teilung der Rollenaufnahme ist darauf zu achten, daß sich immer mindestens drei Rollen innerhalb des Schlauchbettes befinden. Dadurch wird ebenfalls die Kontinuität der Förderung verbessert.

Die Ausbringmenge wird wesentlich auch durch den Innendurchmesser des Schlauches bestimmt. Durch entsprechende Auswahl kann die erfindungsgemäße Schlauchpumpe insbesondere auf die Ausbringung von besonders kleinen Flüssigkeitsmengen bis herunter in den µ-Literbereich eingestellt werden. Die erfindungsgemäße Schlauchpumpe eignet sich dadurch für die wiederholgenaue Abgabe von kleinsten Flüssigkeitsmengen, z.B. Hautklebstoff, wie er in der Hautchirurgie eingesetzt wird.

Vorzugsweise ist der Schlauchpumpe ein Blasendetektor vorgeschaltet, der ein Signal erzeugt, wenn in dem Schlauch der Schlauchpumpe Luft- oder Gasblasen oder kleine feste Fremdkörper gefördert werden. Der Blasendetektor enthält eine Lichtquelle, die einen Lichtstrahl quer durch den Schlauch richtet, wobei der Durchmesser des Lichtstrahls dem Innendurchmesser des Schlauchs entspricht. Der Lichtstrahl trifft auf der anderen Seite des Schlauches auf einen Empfänger. Vorzugsweise wird Infrarotlicht verwendet, wodurch Fremdlichtprobleme weitgehend vermieden werden. Das von dem Empfänger erzeugte Signal wird in der Weise ausgewertet, daß Intensitätsänderungen des durchgelassenen Lichtes festgestellt werden, wobei ein Schwellwert für die Intensitätsänderung eingestellt wird, ab dem ein Signal erzeugt wird, das das Vorliegen einer Luft- oder Gasblase oder eines kleinen festen Fremdkörpers in der Flüssigkeit anzeigt. Dadurch daß nicht die absolute Intensität, sondern nur Intensitätsänderungen gemessen werden, entfällt das Eichen des Blasendetektors auf eine bestimmte Intensität.

Bei der Ausgabe kleinster Flüssigkeitsmengen kann es notwendig sein, die durch die Schlauchwand hindurch stattfindende Verdunstung der Flüssigkeit zu berücksichtigen. Wenn eine bestimmte Flüssigkeitsmenge, z.B. Flüssigkeitstropfen gleicher Größe, in unterschiedlichen Zeitabständen ausgegeben werden sollen, so ist durch entsprechende Wahl des Fördervolumens, d.h. des Schrittwinkels, die seit der Ausgabe des letzten Tropfens verstrichene Zeit zu berücksichtigen und die innerhalb dieser Zeitspanne verdunstete Flüssigkeitsmenge zu kompensieren. Hierbei ist auch zu berücksichtigen, daß ein Schlauch bei seiner erstmaligen Verwendung zunächst eine gewisse Flüssigkeitsmenge absorbiert, so daß die Verdunstung zunächst scheinbar größer ist. Daneben ist auch die gesamte Länge des Schlauches vom Vorratsbehälter bis zur Ausgabeeinrichtung, der Durchmesser des Schlauches, die Wandstärke des Schlauches, das Material des Schlauches und die auszugebende Flüssigkeit (Klebstoff) zu berücksichtigen.

Ein Ausführungsbeispiel der Erfindung wird nachfolgend anhand der Zeichnung erläutert:
- Fig. 1: eine Gesamtansicht des Geräts zur Dosierung und zum Auftragen eines Hautklebstoffes;
- Fig. 2: die in dem Gerät von Fig. 1 enthaltene Schlauchpumpe;
- Fig. 3: im Schnitt mit Blickrichtung längs der Schlauchachse den Blasendetektor und
- Fig. 4: die Schlauchpumpe mit dem eingelegten Schlauch und der Schutzfolie.

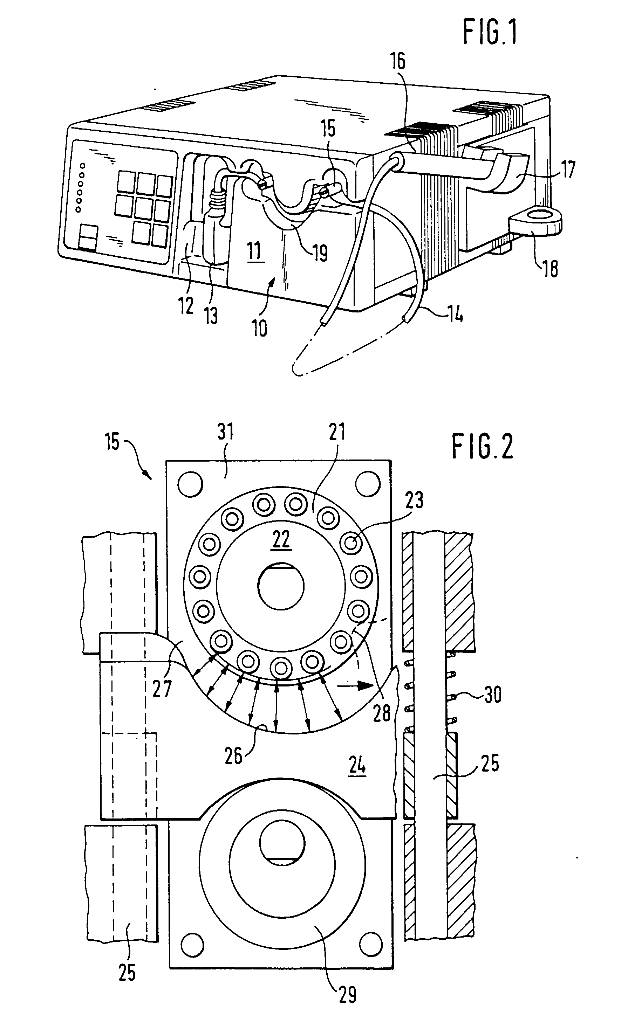

Gemäß Fig. 1 weist das Gerät zum Dosieren eines Hautklebstoffes ein Gehäuse 10, in dem die im einzelnen nicht dargestellte Steuerung und der ebenfalls nicht dargestellte Antriebsmotor für die Schlauchpumpe enthalten sind. In der Frontseite 11 des Gehäuses 10 befindet sich eine Nische 12 zur Aufnahme einer Flasche 13, die den zu dosierenden Hautklebstoff enthält. In die Flasche 13 ist ein Schlauch 14 eingesteckt, der über eine Schlauchpumpe 15 den Klebstoff aus der Flasche 13 zu einem Dosiergriffel 16 fördert, mit dem der Klebstoff aufgetragen wird. Für den Dosiergriffel 16 ist seitlich am Gehäuse 10 ein Holster 17 vorgesehen. Ferner befindet sich auf der gleichen Seite des Gehäuses 10 eine Abtropfschale 18, in die der Klebstoff aus dem Dosiergriffel 16 tropft, wenn sich der Dosiergriffel 16 in seinem Holster 17 befindet. Die Schlauchpumpe 15 befindet sich in dem Gehäuse 10. Um den von der Flasche 13 zu dem Dosiergriffel 16 führenden Schlauch 14 in die Schlauchpumpe 15 einlegen zu können, befindet sich auf der Frontseite 11 des Gehäuses 10 ein Einschnitt 19, der entsprechend dem Verlauf des Schlauches 14 durch die Schlauchpumpe 15 geformt ist, so daß der Schlauch 14 durch den Einschnitt 19 von außen in die Schlauchpumpe 15 eingelegt werden kann. Die leichte Einlegbarkeit und Auswechselbarkeit des Schlauches 14 ist insbesondere bei Hautklebstoff wichtig, da hier häufig ein neuer Schlauch verwendet werden muß.

Die Einzelheiten der Schlauchpumpe 15 sind in Fig. 2 gezeigt. Auf einem Teilkreis 21 einer Rollenaufnahme 22 sind mit einer bestimmten Teilung Rollen 23 frei drehbar gelagert. Die Rollenaufnahme 22 wird durch einen nicht gezeigten Antriebsmotor angetrieben. Im Abstand unterhalb der Rollenaufnahme ist ein Schlauchbett 24 höhenverstellbar gelagert. Das Schlauchbett 24 ist dazu an den beiden Enden auf Führungsstangen 25 verschiebbar gelagert. Die den Rollen 23 zugeordnete Fläche des Schlauchbettes 24 ist konkav und bildet dadurch eine Mulde 26 im Abstand zu der Rollenkreisbahn. Die Mulde 26 ist außermittig gegenüber der Rollenaufnahme 22 angeordnet, und zwar ist sie etwas in Richtung der durch die Drehbewegung der Rollenaufnahme 22 vorgegeben, durch einen Pfeil kenntlich gemachten Förderrichtung versetzt, so daß der Punkt des maximalen Anpreßdruckes der Rollen 23 gegen den Schlauch 14 am Eingang 27 der Mulde 26 liegt. Der Abstand zwischen den Rollen 23 und der Mulde 26 erweitert sich dann in Förderrichtung stetig, so daß sich der für den Schlauch 14 zur Verfügung stehende Raum zwischen den Rollen 23 und der Mulde 26 gebogen keilförmig öffnet. Durch die außermittige Anordnung des Schlauchbettes 24 und den dadurch erreichten gebogenen keilförmigen Öffnungswinkel werden die sinusförmig periodischen Pumpstöße geglättet, so daß eine scheinbar kontinuierliche Förderung des Klebstoffes erreicht wird.

Zur Erzielung einer möglichst kontinuierlichen Förderung trägt auch eine Abstimmung der Anzahl und des Durchmessers der Rollen 23 und des Durchmessers des Teilkreises 21, auf dem die Rollen 23 angeordnet sind, bei. In dem Schlauch 14 bilden sich zwischen aufeinanderfolgenden Rollen 23 sogenannte Rollenkammern 28. Die Rollenkammern 28 dürfen nicht beliebig im Verhältnis zum Teilkreisdurchmesser und dem Rollendurchmesser vergrößert oder verkleinert werden, da sie für die Fördermenge maßgebend sind. Vorteilhalft zur Erzielung eines möglich kontinuierlichen Pumpverhaltens ist dabei insbesondere eine ungerade Anzahl von Rollen 23. Dies folgt aus dem Zusammenhang zwischen Eingangsgröße und Ausgangsgröße bei nichtlinearen Systemen. Die Länge der Mulde 26 und der Abstand der Rollen 23 ist außerdem so zu wählen, daß sich immer mindestens drei Rollen 23 innerhalb der Mulde 26 befinden.

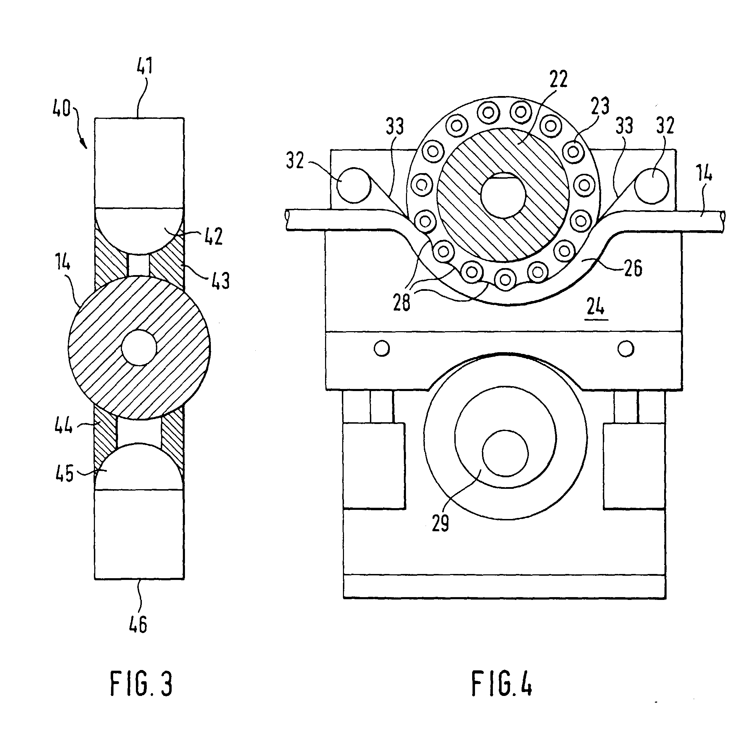

Zweckmäßig wird zwischen die Rollen 23 und den Schlauch 14 eine Schutzfolie 33 gelegt, um die Tangentialkräfte, die durch die Rollen 23 auf den Schlauch 14 ausgeübt werden, abzufangen und dadurch ein Verschieben des Schlauches 14 zu vermeiden. Zusätzlich wird dadurch vermieden, daß der Schlauch 14 an den Rollen 23 kleben bleibt. Die Schutzfolie ist ebenso wie der Schlauch 14 in Fig. 4 gezeigt. Die Schutzfolie ist ein Folienstreifen, dessen Breite etwa der Länge der Rollen 23 entspricht. An ihren Enden ist die Schutzfolie mittels der in Fig. 1 und 4 erkennbaren Spannschrauben 32 gehalten.

Zum Einlegen des Schlauches 14 und gegebenenfalls zum Erneuern der Schutzfolie 33 wird die Schlauchpumpe 15 geöffnet, indem das Schlauchbett 24 etwas von der Rollenaufnahme 22 abgehoben wird. Das Schlauchbett 24 ist dazu auf Führungsstangen 25 mittels einer unterhalb des Schlauchbettes 24 angeordneten Exzenterrolle 29 verschiebbar. In Fig. 2 ist die Schlauchpumpe 15 im geöffneten Zustand und in Fig. 4 im geschlossenen Zustand gezeigt. Das Schlauchbett 24 wird durch Druckfedern 30 gegen die Exzenterrolle 29 vorgespannt. Die Rollenaufnahme 22 und die Exzenterrolle 29 sind auf einer Motorlagerplatte 31 gelagert und das Schlauchbett 24 befindet sich zwischen ihnen.

In Fig. 3 ist ein Blasendetektor 40 dargestellt, der innerhalb des in Fig. 1 gezeigten Gehäuses 10 zwischen der Nische 12 und der Schlauchpumpe 15 eingebaut ist. Der Blasendetektor 40 enthält einen Sender 41 in Form einer Infrarotdiode, eine erste Linse 42, die das Licht des Senders 41 bündelt, eine erste Blende 43, die nur ein Lichtbündel durchläßt, dessen Durchmesser etwas kleiner als der Innendurchmesser des Schlauches 14 ist. Auf der gegenüberliegenden Seite des Schlauches 14 ist eine zweite Blende 44 angeordnet, die von der Seite einfallendes Fremdlicht ausblendet. Hinter der Blende ist eine zweite Linse 45 angeordnet, die das durchgelassene Licht auf einen Empfänger 46 in Form eines Phototransistors fokussiert. Die Wellenlänge des von dem Sender 41 ausgesandten Lichtes richtet sich nach den jeweiligen Verhältnissen, insbesondere der Absorbtion dieser Wellenlänge durch das Material des Schlauches 14 und durch die in dem Schlauch 14 fließende Flüssigkeit sowie nach den Fremdlichtverhältnissen. Im dargestellten Ausführungsbeispiel wird infrarotes Licht einer Wellenlänge von 850-950 nm verwendet, wozu in dem Sender 41 und im Empfänger 46 entsprechende Interferenzfilter eingebaut sind. Wie erwähnt ist der Durchmesser der ersten Blende 43 etwas kleiner als der Innendurchmesser des Schlauches 14. Dadurch wird auch bei einer nicht vollständigen Parallelität des Lichtbündels gewährleistet, daß kein Licht an der im Schlauch 14 vorhandenen Flüssigkeit vorbei zum Empfänger 46 gelangt. Der Durchmesser der zweiten Blende 44 ist zweckmäßig doppelt so groß wie der Innendurchmesser des Schlauches 14.

Der Empfänger 46 erzeugt ein elektrisches Signal, das ungefähr proportional der auf ihn einfallenden Lichtmenge ist. Ist der Schlauch 14 mit der Flüssigkeit gefüllt, so wird ein Teil des Lichts durch die Flüssigkeit absorbiert und erzeugt der Empfänger 46 ein elektrisches Signal. Befinden sich in der Flüssigkeit Luft- oder Gasblasen oder Fremdkörper, so ändert sich während der Bewegung dieser Luft- oder Gasblasen oder Fremdkörper durch den Blasendetektor 40 kurzzeitig die auf den Empfänger 46 auftreffende Lichtmenge, so daß der Blasendetektor einen Signalimpuls erzeugt. Das Signal des Empfängers 46 wird in einer nicht dargestellten elektronischen Schaltung verarbeitet, wobei die Schaltung bei jeder Änderung, wie sie für den Durchgang einer Gasblase oder eines Fremdkörpers charakteristisch ist, ein Ausgangssignal erzeugt. Dadurch, daß die elektronische Schaltung nicht den absoluten Wert des Empfängersignals analysiert, sondern nur auf Änderungen des Detektorsignals reagiert, ist keine Eichung oder Einstellung des Blasendetektors tektors auf einen bestimmten absoluten Signalwert erforderlich.

Der Blasendetektor ist selbstverständlich nicht nur in Verbindung mit der erfindungsgemäßen Schlauchpumpe anwendbar, sondern kann auch in Verbindung mit anderen Geräten oder als selbständige Einheit verwendet werden.

Das in Fig. 1 gezeigte Gerät zur Dosierung und zum Auftragen eines Hautklebstoffes enthält eine Steuereinrichtung, die entsprechend den über ein Tastenfeld 50 eingegebenen Steuersignalen die Schlauchpumpe 15 steuert. Auch die Signale des Blasendetektors 40 werden in der Steuereinrichtung verarbeitet. Hautklebstoff wird von dem Chirurgen im allgemeinen tropfenweise aufgebracht. Die Steuereinrichtung wählt den Schrittwinkel der Rollenaufnahme 20 entsprechend der ausgewählten Tropfengröße. Zum erstmaligen Füllen des Schlauchs 14 kann außerdem auch ein kontinuierlicher Betrieb der Schlauchpumpe 14 angewählt werden. Durch einen (nicht dargestellten) Fußschalter wird die Ausgabe jeweils eines Tropfens der ausgewählten Größe veranlaßt.

Die Steuereinrichtung berücksichtigt dabei auch die zwischen zwei Ausgabevorgängen verstrichene Zeitspanne. Manche Flüssigkeiten, insbesondere Klebstoffe, verdunsten nämlich in geringem Ausmaß durch die Wand des Schlauchs 14 hindurch, wobei das Material des Schlauchs eine wesentliche Rolle spielt. Bei der Dosierung besonders kleiner Flüssigkeitsmengen ist es daher notwendig, die durch den Schlauch 14 hindurchdiffundierte Flüssigkeitsmenge zu kompensieren, indem bei dem nächsten Dosiervorgang dieser Flüssigkeitsverlust ausgeglichen wird. Zusätzlich zeigt die Steuereinrichtung durch optische oder akustische Signale es auch an, wenn die Verarbeitungszeit des Klebstoffes abgelaufen ist.

Die Steuereinrichtung steuert die Ausgabeeinrichtung dabei so, daß die zwischen zwei Ausgabevorgängen verstrichene Zeitspanne berücksichtigt, um die in dieser Zeitspanne durch Verdunstung durch den Schlauch oder Absorption im Schlauch auftretende Flüssigkeitsverluste zu kompensieren.

## Patentansprüche

1. Schlauchpumpe
mit einem Schlauchbett (24) und
mit einer Anzahl Rollen (23), die drehbar auf einer drehangetriebenen Rollenaufnahme (22) auf einen Teilkreis (21) gelagert sind und durch die Rollenaufnahme (22) auf einer Kreisbahn im Abstand von dem Schlauchbett (24) bewegt werden,
wobei der Abstand so gewählt ist, daß die Rollen (23) einen Schlauch (14) der zwischen dem Schlauchbett (24) und der Kreisbahn der Rollen (23) liegt, auf einem Teil ihrer Kreisbahn zusammendrücken können,
**dadurch gekennzeichnet ,**
daß das Schlauchbett (24) versetzt gegenüber der Kreisbahn der Rollen (23) angeordnet ist, so daß der Abstand zwischen dem Schlauchbett (24) und der Kreisbahn der Rollen (23) in Bewegungsrichtung der Rollen (23) zunimmt.

2. Schlauchpumpe nach Anspruch 1, dadurch gekennzeichnet, daß das Schlauchbett (24) außerhalb des Teilkreises (21) der Rollenaufnahme (22) angeordnet ist und daß die der Rollenaufnahme (22) zugewandte Oberfläche eine konkave Mulde (26) ist.

3. Schlauchpumpe nach Anspruch 2, dadurch gekennzeichnet, daß das Schlauchbett (24) durch Federkraft gegen eine Exzenterrolle (29) vorgespannt ist und durch Drehen der Exzenterrolle (29) verschiebbar ist.

4. Schlauchpumpe nach einem der Ansprüche 1 bis 3, dadurch gekennzeichnet, daß auf der Rollenaufnahme (22) eine ungerade Anzahl von Rollen (23) angeordnet ist.

5. Schlauchpumpe nach einem der Ansprüche 1 bis 4, dadurch gekennzeichnet, daß sich immer mindestens drei Rollen (23) innerhalb des Schlauchbettes (24) befinden.

6. Schlauchpumpe nach einem der Ansprüche 1 bis 5, dadurch gekennzeichnet, daß zwischen den Rollen (23) und dem Schlauch (14) eine Schutzfolie (33) angeordnet ist.

7. Schlauchpumpe nach einem der Ansprüche 1 bis 6, gekennzeichnet durch eine Steuereinrichtung, die zur Ausgabe einzelner Tropfen bestimmter Größe auf ein externes Signal hin den Drehantrieb so steuert, daß die Rollenaufnahme (22) sich um einen bestimmten Drehwinkel dreht, wobei der Drehwinkel von der gewählten Tropfengröße und zur Kompensation von inzwischen eingetretenen Verdunstungsverlusten auch von der seit der letzten Ausgabe eines Tropfens verstrichenen Zeit abhängt.

8. Schlauchpumpe nach Anspruch 7, dadurch gekennzeichnet, daß die Steuereinrichtung unter Berücksichtigung der zwischen zwei Ausgabevorgängen verstrichenen Zeitspanne und der innerhalb dieser Zeitspanne durch Verdunstung oder Absorption in den Förderschläuchen aufgetretenen Flüssigkeitsverluste den Drehantrieb so steuert, daß diese Verluste durch Vergrößerung des Drehwinkels kompensiert werden.

9. Schlauchpumpe nach einem der Ansprüche 1 bis 8, mit einem Blasendetektor (40) zum Erkennen von Gasblasen oder Fremdkörpern in einer durch einen Schlauch (14) geförderten Flüssigkeit mit einem Sender (41), der einen gebündelten Lichtstrahl durch den Schlauch (14) richtet, mit einem Empfänger (46) zum Empfang des Lichtstrahls nach dem Durchgang durch den Schlauch (14) und mit einer Steuereinrichtung, dadurch gekennzeichnet, daß die Steuereinrichtung das von dem Empfänger (46) abgegebene Signal in der Weise verarbeitet, daß bei einer Änderung der von dem Empfänger (46) empfangenen Lichtmenge ein das Vorhandensein von Blasen oder Fremdkörpern anzeigendes Warnsignal erzeugt wird.

10. Schlauchpumpe nach Anspruch 9, dadurch gekennzeichnet, daß zwischen dem Sender (41) und dem Schlauch (14) eine Blende (43) vorhanden ist, deren Öffnung etwas kleiner als der Innendurchmesser des Schlauchs (14) ist.

## Claims

1. Hose pump comprising
a hose bed (24) and
a number of rollers (25) which are housed in rotatable manner on a rotary-driven roller holder (22) on a pitch circle (21) and are moved by the roller holder (22) on an orbital path at a distance from the hose bed (24),
whereby the distance is chosen such that the rollers (22) can compress a hose (14), which lies between the hose bed (24) and the orbital path of the rollers (22), on one part of their orbital path,
characterized in that
the hose bed (24) is arranged offset vis-a-vis the orbital path of the rollers (22) so that the distance between the hose bed (24) and the orbital path of the rollers (22) increases in the direction of movement of the rollers (22) .

2. Hose pump according to claim 1, characterized in that thehose bed (24) is arranged outside the pitch circle (21) of the roller holder (22) and that the surface facing the roller holder (22) is a concave trough (26).

3. Hose pump according to claim 2, characterized in that the hose bed (24) is pre-tensioned by spring tension against an eccentric roller (29) and is displaceable by rotating the eccentric roller (29).

4. Hose pump according to one of claims 1 to 3, characterized in that an odd number of rollers is arranged on the roller holder (22) .

5. Hose pump according to one of claims 1 to 4, characterized in that at least three rollers (23) are always positioned within the hose bed (24).

6. Hose pump according to one of claims 1 to 5, characterized in that a protective film (33) is arranged between the rollers (23) and the hose (14).

7. Hose pump according to one of claims 1 to 6, characterized by a control system which, for the dispensing of individual drops of a certain size following an external signal, controls the rotary drive in such a way that the roller holder (22) rotates by a certain angle of rotation, whereby the angle of rotation depends on the chosen drop size and also on the time elapsed since the last dispensing of a drop for the compensation of evaporation losses which have taken place in the meantime.

8. Hose pump according to claim 7, characterized in that the control system, taking into account the time span elapsed between two dispensing processes and the losses of liquid which have occurred within this time span through evaporation or absorption in the delivery hoses, controls the dispensing device such that those losses are compensated by increasing the delivery quantity.

9. Hose pump according to one of claims 1 to 8, comprising a bubble detector (40) for detecting gas bubbles or foreign bodies in a liquid conveyed through a hose (14), having a transmitter (41) which directs a focused light beam through the hose (14), having a receiver (46) for receiving the light beam after the passage through the hose (14) and having a control system, characterized in that the control system processes the signal given off by the receiver (46) in such a way that a warning signal indicating the presence of bubbles or foreign bodies is generated when the quantity of light received by the receiver (46) changes.

10. Hose pump according to claim 9, characterized in that there is a screen (43) between the transmitter (41) and the hose (14), the aperture of which is somewhat smaller than the internal diameter of the hose (14).

## Revendications

1. Pompe tubulaire comprenant
un lit (24) de tuyau
un certain nombre de rouleaux (23), qui sont placés sur un cercle divisé (21) en pouvant tourner sur un organe (22) récepteur de rouleaux entraîné en rotation et sont déplacés par l'organe récepteur (22) de rouleaux sur une trajectoire circulaire à distance du lit (24) de tuyau,
la distance étant choisie de manière que les rouleaux (23) puissent comprimer sur une partie de leur trajectoire circulaire un tuyau (14) situé entre le lit (24) de tuyau et la trajectoire circulaire des rouleaux (23),
caractérisé en ce que
le lit (24) de tuyau est disposé décalé par rapport à la trajectoire circulaire des rouleaux (23), de telle manière que la distance entre le lit (24) de tuyau et la trajectoire circulaire des rouleaux (23) augmente dans le sens du mouvement des rouleaux (23).

2. Pompe tubulaire selon la revendication 1, caractérisée en ce que le lit (24) de tuyau est disposé à l'extérieur du cercle divisé (21) de l'organe (22) récepteur de rouleaux et que la surface tournée vers l'organe (22) récepteur de rouleaux est une cuvette concave (26).

3. Pompe tubulaire selon la revendication 2, caractérisée en ce que le lit (24) de tuyau est mis en précontrainte par force élastique contre un rouleau excentrique (29) et peut être déplacé par rotation du rouleau excentrique (29).

4. Pompe tubulaire selon l'une des revendications 1 à 3, caractérisée en ce qu'un nombre impair de rouleaux (23) est placé sur l'organe (22) récepteur de rouleaux.

5. Pompe tubulaire selon l'une des revendications 1 à 4, caractérisée en ce qu'il se trouve toujours au moins trois rouleaux (23) à l'intérieur du lit (24) de tuyau.

6. Pompe tubulaire selon l'une des revendications 1 à 5, caractérisée en ce qu'une feuille de protection (33) est placée entre les rouleaux (23) et le tuyau (14).

7. Pompe tubulaire selon l'une des revendications 1 à 6, caractérisée par un dispositif de commande qui, pour la distribution de gouttes séparées d'une grosseur déterminée d'après un signal extérieur, commande le mouvement de rotation de telle manière que l'organe (22) récepteur de rouleaux tourne d'un angle de rotation déterminé, l'angle de rotation dépendant de la grosseur de goutte choisie et, pour la compensation des pertes par évaporation survenues entre-temps, également du temps écoulé depuis la dernière distribution d'une goutte.

8. Pompe tubulaire selon la revendication 7, caractérisée en ce que le dispositif de commande commande l'entraînement en rotation en prenant en considération l'intervalle de temps écoulé entre deux phénomènes de distribution et les pertes de liquide par évaporation ou absorption survenues dans cet intervalle de temps dans les tuyaux de transport de telle manière que ces pertes soient compensées par l'agrandissement de l'angle de rotation.

9. Pompe tubulaire selon l'une des revendications 1 à 8, comportant un détecteur de bulles (40) destiné à la reconnaissance de bulles de gaz ou de corps étrangers dans un liquide transporté par un tuyau (14), un émetteur (41) qui dirige un faisceau lumineux à travers le tuyau (14), un récepteur (46) destiné à recevoir le rayon lumineux après traversée du tuyau (14) et un dispositif de commande, caractérisée en ce que le dispositif de commande traite le signal émis par le récepteur (46) de telle manière qu'une variation de la quantité de lumière reçue par le récepteur (46) déclenche une alarme indiquant la présence de bulles ou de corps étrangers.

10. Pompe tubulaire selon la revendication 9, caractérisée en ce que, entre l'émetteur (41) et le tuyau (14), il y a un diaphragme (43) dont l'ouverture est un peu plus petite que le diamètre intérieur du tuyau (14).
